# EUROPÄISCHE PATENTANMELDUNG

(11) **EP 2 774 677 A2**
(43) Veröffentlichungstag der Anmeldung: **10.09.2014**
(21) Anmeldenummer: 14154953.5
(22) Anmeldetag: 13.02.2014
(51) Int. Cl.: B01L 3/00

(54) **Vorrichtung zur Durchführung von chemischen und/oder biochemischen Prozessen**

(30) Priorität: 05.03.2013 DE 102013203682
(71) Anmelder: ROBERT BOSCH GMBH, 70442 Stuttgart (DE)
(72) Erfinder: Kloke, Arne, 79102 Freiburg (DE); Steigert, Juergen, 70176 Stuttgart (DE)

(57) **Zusammenfassung**

Bei einer Vorrichtung (20) zur Durchführung von chemischen und/oder biochemischen Prozessen umfasst die Vorrichtung (20) wenigstens zwei axial übereinander angeordnete Körper (21, 22, 23) mit jeweils wenigstens einer Kavität (28, 29, 30, 31, 33, 34). Die Körper (21, 22, 23) sind in Abhängigkeit von einer Zentrifugalkraft oder einer gleichwirkenden Kraft gegeneinander verdrehbar. Erfindungsgemäß ist in wenigstens einer Kavität (31) eine Membran (111) vorgesehen, deren Durchlässigkeit für Flüssigkeiten von der wirkenden Zentrifugalkraft oder der gleichwirkenden Kraft abhängig ist.

## Beschreibung

Die vorliegende Erfindung betrifft eine Vorrichtung zur Durchführung von chemischen und/oder biochemischen Prozessen sowie eine Verwendung eines Reaktionsbehältnisses.

### Stand der Technik

Die Durchführung von biochemischen oder chemischen Prozessen, beispielswiese im Zusammenhang mit der Aufreinigung bestimmter Moleküle und/oder mit der Analyse und Charakterisierung bestimmter Moleküle, basiert im Wesentlichen auf der Handhabung von Flüssigkeiten. Herkömmlicherweise werden hierfür verschiedene Hilfsmittel, insbesondere Pipetten und verschiedene Reaktionsgefäße, eingesetzt, um bei einer manuellen Handhabung unter Zuhilfenahme verschiedener Laborgeräte die verschiedenen Prozesse durchführen zu können. Für viele Reaktionen sind bereits Automatisierungen verfügbar, wobei beispielsweise Pipettierroboter oder andere Spezialgeräte zum Einsatz kommen. Weiterhin können mit sogenannten Lab-on-a-Chip-Systemen viele biochemische Prozesse in voll automatisierter Weise durchgeführt werden. Es handelt sich hierbei um mikrofluidische Systeme, welche die gesamte Funktionalität eines makroskopischen Labors auf einem nur etwa plastikkartengroßen Kunststoffsubstrat vereinigen. Neben dem Kunststoffsubstrat mit verschiedenen Kanälen, Reaktionskammern usw. sind vorgelagerten Reagenzien und verschiedene aktive Komponenten, wie beispielsweise Ventile oder Pumpen, sowie weitere Aktuations-, Detektions- und Steuereinheiten erforderlich.

Für viele chemische oder biochemische Prozesse wird eine Zentrifugation eingesetzt. Durch die hierbei erzeugte Zentrifugalkraft kann eine Stofftrennung aufgrund eines Dichteunterschiedes zwischen den verschiedenen Komponenten eines Gemisches durchgeführt werden. Weiterhin ermöglicht die Zentrifugation einen Transport von Flüssigkeiten von einer radial weiter innenliegenden Prozessstufe zu einer radial weiter außenliegenden Prozessstufe.

Die deutsche Offenlegungsschrift DE 10 2010 003 223 A1 beschreibt ein System, das eine Vorrichtung umfasst, die zum Einsetzen in einen zentrifugalen Rotor vorgesehen ist. Hierbei sind zwei oder mehr revolverartige Körper axial übereinander angeordnet. Die Revolver beinhalten dabei ein oder mehrere Kavitäten, insbesondere Reaktionskammern, Kanäle und gegebenenfalls weitere Strukturen für die Durchführung von Prozessen. Ein Beschleunigungswechsel der Zentrifuge aktiviert eine integrierte Mechanik, die nach Art einer Kugelschreibermechanik funktioniert. Infolge der Zentrifugalkraft bewegen sich die Körper radial nach außen, wobei die Körper mittels einer Verzahnung und einem integrierten Rückstellmittel gegeneinander verdreht werden. Einzelne Kavitäten können hierdurch miteinander verschaltet werden. Darüber hinaus ist ein orientierungsabhängiges Öffnen von einzelnen Kavitäten oder Gefäßen eines Körpers möglich, wobei eine Seite des Gefäßes beispielsweise mit einer durchstechbaren Folie versehen ist. Mit Hilfe eines Dorns auf dem anderen Körper wird die Folie durch die Bewegung der Körper gegeneinander durchstoßen. Auf diese Weise kann eine kontrollierte Fluidführung in der Vorrichtung erreicht werden. Beispielsweise kann eine Fluidführung von Vorlagerungskammern über zwischengeschaltete Prozessierungskammern bis hin zu Auffangkavitäten für die prozesszierten Flüssigkeiten realisiert werden.

Viele biochemische Methoden nutzen kleine Kügelchen, sogenannte Beads, um beispielsweise eine Aufreinigung von bestimmten Molekülen durchzuführen. An oder auf die Beads können bestimmte Wechselwirkungspartner eines aufzureinigenden Moleküls gekoppelt oder aufgetragen werden. Bei einer Inkubation dieser Beads mit einem Gemisch, das das aufzureinigende Molekül enthält, bindet das Molekül an den Wechselwirkungspartner und ist damit an die Beads gekoppelt. Die Beads können dann beispielsweise durch Sedimentation oder aufgrund von magnetischen Eigenschaften von der übrigen Lösung isoliert werden, so dass sehr effizient die Aufreinigung des Moleküls erfolgen kann.

Die internationale Patentanmeldung WO 01/85341 A1 beschreibt eine mikrofluidische Vorrichtung in Form eines Chips, bei dem ein Kompartiment mit Beads vorgesehen ist, die mit einem Filter zurückgehalten werden. Dieser Chip kann für Bead-basierte Aufreinigungsverfahren eingesetzt werden.

### Offenbarung der Erfindung

### Vorteile der Erfindung

Die erfindungsgemäße Vorrichtung ist zur Durchführung, insbesondere zur automatisierten Durchführung, von chemischen und/oder biochemischen Prozessen vorgesehen, beispielsweise für eine Aufreinigung von Proteinen und/oder von genetischem Material, insbesondere von DNA oder RNA. Für die Durchführung der Prozesse wird ein bestimmter Fluidfluss in der Vorrichtung vorgegeben, wobei hierfür eine Zentrifugalkraft ausgenutzt wird. Die Vorrichtung kann zum Einsetzen in einen Rotor einer Zentrifuge vorgesehen sein. Die Vorrichtung umfasst wenigstens zwei axial übereinander angeordnete Körper mit jeweils wenigstens einer Kavität. In einem ersten Körper können beispielsweise mehrere Kavitäten vorgesehen sein, die für verschiedene Reagenzien, Lösungen oder Gemische vorgesehen sind, die für den jeweiligen Prozess benötigt werden. In dem anderen Körper können ein oder mehrere Kavitäten vorgesehen sein, die beispielsweise als Reaktionsräume dienen. Die wenigstens zwei Körper sind in Abhängigkeit von einer Zentrifugalkraft oder einer gleichwirkenden Kraft gegeneinander verdrehbar, so dass in vorgebbarer Weise jeweils eine Kavität des einen Körpers mit einer Kavität des anderen Körpers in räumliche Nähe zueinander gebracht werden können, so dass ein Fluidfluss stattfinden kann. Statt einem Einsetzen der Vorrichtung in den Rotor einer Zentrifuge und dem Ausüben einer Zentrifugalkraft kann die Vorrichtung beispielsweise auch mit Druckluft beaufschlagt werden. Hierdurch kann ebenfalls ein Verdrehen der Körper erreicht werden, wobei die Vorteile eines stationären Systems genutzt werden können, beispielsweise können verschiedene Parameter einfacher als in einer Zentrifuge eingestellt werden. Wenn im Folgenden Zentrifugalkräfte erwähnt werden, sind die Zentrifugalkräfte im Prinzip durch gleichwirkende Kräfte, die beispielsweise durch Druckluft ausgelöst werden, austauschbar. Erfindungsgemäß ist in wenigstens einer Kavität eines Körpers eine Membran vorgesehen, deren Durchlässigkeit für Flüssigkeiten von der wirkenden Zentrifugalkraft oder der gleichwirkenden Kraft abhängig ist. Durch den Einsatz einer solchen Membran in der Vorrichtung wird eine Ventilwirkung erreicht. Das heißt, der Fluidfluss durch die Kavität mit der Membran erfolgt in Abhängigkeit von der angelegten Zentrifugalkraft oder der gleichwirkenden Kraft. Eine Flüssigkeit kann in dieser Kavität also mehrfach zurückgehalten und freigesetzt werden. Darüber hinaus ermöglicht die Membran in der erfindungsgemäßen Vorrichtung, dass in dieser Kavität eine Inkubation einer Probe bzw. einer Flüssigkeit mit einer Festphase durchgeführt wird. Hierfür können beispielsweise Beads in der Kavität enthalten sein, die durch ihre spezifischen Eigenschaften insbesondere eine Bindung beispielsweise eines aufzureinigenden Proteins oder eines anderen Moleküls erlauben.

Aus der deutschen Offenlegungsschrift DE 10 2010 003 223 A1 ist eine Vorrichtung mit zwei axial übereinander angeordneten Körpern mit jeweils wenigstens einer Kavität bekannt, wobei die Körper gegeneinander verdrehbar sind. Bei dieser Vorrichtung ist ein positionsabhängiges Öffnen von wenigstens einer Kavität vorgesehen, wobei insbesondere mithilfe eines Dorns, der an einem Körper angeordnet ist, eine Folie, die einen Verschluss einer anderen Kavität bildet, aufgerissen oder aufgestochen wird und so Flüssigkeit freigesetzt wird. Die erfindungsgemäße Lösung hat demgegenüber den Vorteil, dass das Freisetzen der Flüssigkeit bzw. das Öffnen einer Kavität mehrfach durchgeführt werden kann bzw. reversibel ist. Je nach angelegter Zentrifugalkraft oder gleichwirkender Kraft kann Flüssigkeit aus einer Kavität freigesetzt werden oder die Flüssigkeit verbleibt in der Kavität.

Durch die Ventilwirkung der erfindungsgemäß eingesetzten Membran kann die erfindungsgemäße Vorrichtung sehr variabel eingesetzt werden und für ganz verschiedene durchzuführende Protokolle angepasst und eingerichtet werden. Insbesondere kann beispielsweise ein mehrfacher Probenauftrag und Durchlauf von verschiedenen Puffern auf eine Bead-Matrix durchgeführt werden, wobei sich die entsprechenden Beads in der Kavität mit der Membran befinden. Durch eine erfindungsgemäße Verwendung der Membran beispielsweise als Bodenverschluss von Reagenzmittelvorhaltungskavitäten kann durch Anlegen von entsprechenden Zentrifugalkräften oder gleichwirkenden Kräften eine Flüssigkeit mehrfach, im Prinzip beliebig oft, aus der Kavität freigesetzt und entlang des vorgesehenen fluidischen Pfades beispielsweise in eine Reaktionskavität überführt werden.

Gegenüber einer Vorrichtung, bei der Flüssigkeit durch mechanische Zerstörung einer Verschlussfolie freigesetzt wird, hat die erfindungsgemäße Vorrichtung darüber hinaus den Vorteil, dass die Membran gemäß der Erfindung die Wirkung eines mehrfach verschließbaren Ventils zeigt, so dass bei entsprechender Ansteuerung bzw. Auslegung der Vorrichtung und des Zentrifugationsprotokolls die freigesetzte Flüssigkeit für eine bestimmte Zeit in einem definierten Raum zurückgehalten werden kann und dann wieder entlang des vorgesehenen fluidischen Pfades weitergeleitet wird. Es können somit ohne Weiteres mehrere Flüssigkeiten miteinander inkubiert werden. Weiterhin ist die Inkubation der Flüssigkeit mit einer Festphase möglich, wobei die Festphase beispielsweise von Beads gebildet wird, die in an sich bekannter Weise eine sehr effektive Aufreinigung eines Zielmoleküls aus einer Lösung erlauben. Somit können insbesondere Bead-basierte Aufreinigungsprotokolle mithilfe der erfindungsgemäßen Vorrichtung ohne zusätzlichen apparativen Aufwand durchgeführt werden. Ein großer Vorteil ist, dass vollständig auf kostenintensive magnetische Beads verzichtet werden kann, die entsprechende magnetische Spezialvorrichtungen zur Abtrennung von der Lösung erfordern würden.

Die Ventilwirkung der Membran wird durch die Druckabhängigkeit der Membrandurchlässigkeit erzielt. Zweckmäßigerweise wird die Membran so ausgewählt, dass der Druck der Flüssigkeitssäule bei geringen Zentrifugalkräften oder gleichwirkenden Kräften unterhalb des Druckes liegt, der für eine Durchdringung der Membran mit der jeweiligen Flüssigkeit erforderlich ist. Bei geringer Zentrifugalkraft ist das "Ventil" folglich geschlossen. Bei einer Erhöhung der Zentrifugalkraft nimmt der Druck zu, so dass die Flüssigkeit durch die Membran hindurch dringt. Bei erhöhter Zentrifugalkraft ist das "Ventil" damit offen bzw. durchlässig. Die Druckabhängigkeit der Durchlässigkeit kann je nach Anwendungsfall eingerichtet und angepasst werden, indem eine geeignete Membran eingesetzt wird. Es kann beispielsweise bevorzugt sein, dass die Membran bei einer Gravitationsbeschleunigung von < 100 g für Flüssigkeiten undurchlässig und bei einer Gravitätsbeschleunigung von > 100 g für Flüssigkeiten durchlässig ist. Hierbei bezeichnet g die Erdbeschleunigung. Die Porengröße der Membran kann beispielsweise so gewählt werden, dass die Porengröße 10 µm oder kleiner, insbesondere 5 µm oder kleiner, oder 1 µm oder kleiner ist. Bei dem Einsatz von Beads in der Kavität mit der Membran ist der Porendurchmesser der Membran zweckmäßigerweise kleiner als der Durchmesser der Beads.

Neben der Porosität, der Porengröße und der Porengeometrie hängen die Durchlässigkeitseigenschaften einer Membran auch von dem Material der Membran ab. Die gewählten Materialien für die Membran können je nach Anwendungsfall hydrophile oder hydrophobe Eigenschaften aufweisen. Geeignet sind beispielsweise Silicamembranen oder Polymermembranen. Insbesondere für Proteinanwendungen sind Membranen aus PVDF (Polyvinylidenfluorid) aufgrund ihrer Absorptionseigenschaften geeignet. Weiterhin können auch die Benetzungseigenschaften der Membran eine Rolle spielen, so dass bei der Wahl einer geeigneten Membran auch die Eigenschaften der Flüssigkeit, die durch die Membran zurückgehalten bzw. ausgespült werden soll, einfließen können.

Die Membran in der erfindungsgemäßen Vorrichtung kann beispielsweise auf einer porösen Trägerstruktur gelagert sein, beispielsweise einer Glasfritte. Die Membran kann mit mechanischen Hilfsmitteln fixiert und/oder abgedichtet sein, beispielsweise mit einem Dichtungsring.

Bei der erfindungsgemäßen Vorrichtung ist für die Verdrehung der Körper gegeneinander insbesondere ein Eingriff von Führungsfedern des einen Körpers in eine Profilzahnreihe des anderen Körpers vorgesehen. Der Begriff "Feder" wird erfindungsgemäß im Sinne einer Feder in einer Nut-Feder-Verbindung verstanden. Weiterhin ist ein Rückstellmittel, insbesondere eine Feder, vorgesehen, die entgegen der Zentrifugalkraft oder der gleichwirkenden Kraft wirkt. Die Körper sind in der Vorrichtung so angeordnet, dass sie bei wirkender Zentrifugalkraft radial nach außen innerhalb eines umgebenden Hüllkörpers verschoben werden. Bei einem Anlegen von Druckluft im oberen Bereich der Vorrichtung werden die Körper innerhalb des Hüllkörpers nach unten verschoben. Einer der Körper ist hierbei beispielsweise durch Fixierungsmittel so arretiert, dass er sich nicht verdrehen kann, aber dennoch radial bzw. nach unten beweglich ist. Der andere Körper verdreht sich durch den Eingriff der Führungsfedern in eine Profilzahnreihe des arretierten Körpers nach Art einer Kugelschreibermechanik. Auf diese Weise werden die verschiedenen Kavitäten der einzelnen Körper in geführter Weise in räumliche Nähe zueinander gebracht, so dass der Fluidfluss entsprechend geschaltet werden kann und der Prozess auf diese Weise geführt werden kann.

Die Erfindung umfasst weiterhin die Verwendung eines Reaktionsbehältnisses mit einer darin angeordneten Membran, deren Durchlässigkeit für Flüssigkeiten von einer wirkenden Zentrifugalkraft oder einer gleichwirkenden Kraft abhängig ist, für die Durchführung von chemischen und/oder biochemischen Prozessen, insbesondere für die Durchführung einer automatisierten Reinigung von Proteinen und/oder von genetischem Material. Die erfindungsgemäße Verwendung ist insbesondere für den Einsatz von Bead-basierten Aufreinigungsprotokollen geeignet, wobei die Membran eine Rückhaltefunktion für die Beads ausübt. Bead-basierte Aufreinigungsprotokolle können erfindungsgemäß mit sehr geringem apparativem Aufwand durchgeführt werden.

Durch die bereits beschriebene Ventilwirkung der in dem Reaktionsbehältnis angeordneten Membran kann das Reaktionsbehältnis in sehr vorteilhafter Weise für die verschiedensten Reinigungsprotokolle von biologischen oder anderen Molekülen eingesetzt werden. In besonders bevorzugter Weise wird das Reaktionsbehältnis als Teil der bereits beschriebenen erfindungsgemäßen Vorrichtung verwendet.

Weitere Merkmale und Vorteile der Erfindung ergeben sich aus der nachfolgenden Beschreibung von Ausführungsbeispielen im Zusammenhang mit den Zeichnungen. Hierbei können die einzelnen Merkmale jeweils für sich oder in Kombination miteinander verwirklicht sein.

### Kurze Beschreibung der Zeichnungen

In den Zeichnungen zeigen:
- Fig. 1: schematische Schnittansicht einer Kavität mit Membran als Ausschnitt aus einer bevorzugten Ausführungsform einer erfindungsgemäßen Vorrichtung und
- Fig. 2: schematische Schnittansicht einer bevorzugten Ausgestaltung einer erfindungsgemäßen Vorrichtung.

### Beschreibung von Ausführungsbeispielen

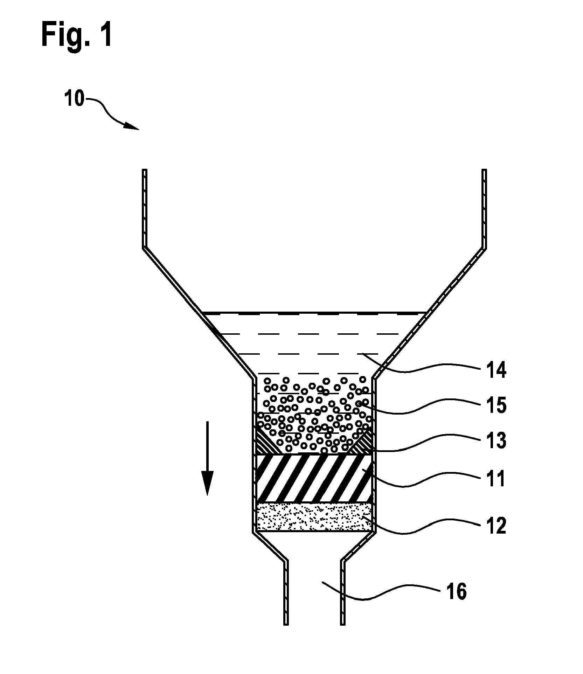

**Fig. 1** zeigt eine Kavität mit einer Membran als erfindungswesentliches Element einer erfindungsgemäßen Vorrichtung. Die Kavität ist als Reaktionsbehältnis 10 ausgestaltet, wobei in Richtung des Fluidflusses, der durch einen Pfeil angedeutet ist, eine trichterförmige Verjüngung des Reaktionsbehältnisses 10 vorgesehen ist. In einem zylinderförmigen Abschnitt innerhalb der Verjüngung des Reaktionsbehältnisses 10 ist eine Membran 11 angeordnet, die den Querschnitt des Behältnisses an dieser Stelle ausfüllt. Unterhalb der Membran 11 befindet sich eine poröse Träger- oder Stützstruktur 12, auf der die Membran 11 stabil gelagert ist. Als poröse Stützstruktur 12 können beispielsweise Glasfritten eingesetzt werden. Als Material für die Membran 11 eignen sich vor allem Materialien, die eine geringe Absorption gegenüber der aufzureinigenden Substanz aufweisen. Im Falle einer Proteinaufreinigung können beispielsweise PVDF-Membranen eingesetzt werden. An den Berührungsstellen der Membran 11 mit der Wandung des Reaktionsgefäßes ist ein Dichtungsring 13 vorgesehen, um einen ungewollten Fluidfluss zu vermeiden. Oberhalb der Membran 11 ist eine Flüssigkeitssäule 14 dargestellt. In der Flüssigkeit sind Beads 15 enthalten. Der Porendurchmesser der Membran 11 ist zweckmäßigerweise kleiner als die Abmessungen der zurückzuhaltenden Stoffe, also insbesondere der Beads 15. Der Porendurchmesser liegt vorzugsweise unterhalb von 10 µm. Besonders geeignet ist beispielsweise ein Porendurchmesser von < 1 µm, zum Beispiel 0,45 µm. Als Beads können beispielsweise Silicabeads, Nickelbeads, Polymerbeads oder Glasbeads eingesetzt werden. Je nach Anwendungsfall, beispielsweise je nach aufzureinigendem Molekül, werden bestimmte Oberflächeneigenschaften der Beads genutzt. Es können beispielsweise native Beads eingesetzt werden, die aufgrund ihrer materialimmanenten Bindungseigenschaften eine Affinität zu dem jeweiligen aufzureinigenden Molekül aufweisen. In anderen Fällen können zusätzliche Bindungschemien eingesetzt werden. Beispielsweise können bestimmte Wechselwirkungspartner der zu isolierenden Zielmoleküle an die Beads gekoppelt werden, um so eine Anbindung des Ziel moleküls an die Beads zu erreichen.

Die Beads 15 werden direkt auf der Membran 11 (Filtermembran) gelagert. Je nach einwirkender Zentrifugalkraft oder gleichwirkender Kraft ist die Membran für Flüssigkeiten undurchlässig oder durchlässig. Wenn also eine entsprechende Zentrifugalkraft oder gleichwirkende Kraft ausgeübt wird, dringt die Flüssigkeit 14 durch die Membran 11 und den ohnehin durchlässigen Träger 12 und wird entlang der Fluidführung 16 abgeleitet. Die Beads 15 werden dabei zurückgehalten. Hierbei entspricht die Richtung des Fluidflusses, der durch den Pfeil angedeutet ist, auch der Richtung der wirkenden Zentrifugalkraft oder der gleichwirkenden Kraft.

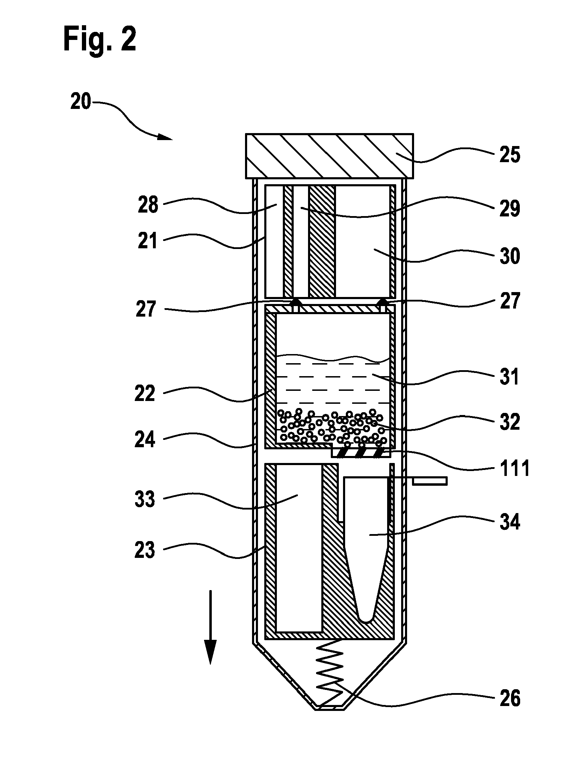

Das erfindungsgemäße "Membranventil" ist für verschiedene Anwendungen, insbesondere im Zusammenhang mit einer automatisierten Proteinaufreinigung und/oder einer DNA- oder RNA-Aufreinigung geeignet. Es kann insbesondere in zentrifugalen Vorrichtungen verwendet werden, vor allem in einem System, bei dem zwei oder mehr axial übereinander angeordnete Körper mit jeweils einer Kavität vorhanden sind und wobei der Fluidfluss für eine automatisierte Durchführung eines Aufreinigungsprotokolls durch eine Drehung der Körper gegeneinander realisiert wird. **Fig. 2** zeigt ein solches zentrifugales System 20 als Beispiel für eine erfindungsgemäße Vorrichtung. In dieser Ausführungsform sind drei Körper 21, 22 und 23 vorgesehen, die gegeneinander verdrehbar sind. Die Körper 21, 22 und 23 können auch als Revolver bezeichnet werden, da sie jeweils ein oder mehrere Kavitäten aufweisen, die sich in unterschiedlichen Positionen zueinander befinden können. Der Revolver 21 und der Revolver 23 sind hierbei durch entsprechende Führungsmittel innerhalb eines umgebenden Hüllkörpers 24 arretiert, so dass sich ausschließlich der Revolver 22 in Bezug auf den Revolver 21 und den Revolver 23 verdrehen kann. Der Hüllkörper 24 entspricht von der äußeren Form her einem üblichen Zentrifugationsröhrchen, beispielsweise mit einem Volumen von 50 ml. Der Hüllkörper 24 ist mit einem Deckel 25 verschließbar. Innerhalb des Hüllkörpers 24 sind die Revolver 21 und 22 und gegebenenfalls auch der Revolver 23 derart angeordnet, dass sie in radialer Richtung, angedeutet durch einen Pfeil, infolge einer wirkenden Zentrifugalkraft verschieblich sind. Das heißt, bei einer wirkenden Zentrifugalkraft werden die Körper 21, 22 und 23 in Pfeilrichtung, das heißt in dieser Darstellung nach unten, verschoben. Diese Verschiebung wirkt entgegen der Rückstellkraft eines Rückstellmittels 26, das in dieser Darstellung als Feder 26 im unteren, konisch zulaufenden Ende des Hüllkörpers 24 vorgesehen ist. Bei nachlassender Zentrifugalkraft bewirkt die Feder 26, dass die Revolver 21, 22 und 23 wieder zurück in ihre Ausgangsposition bewegt werden. Durch geeignete, hier nicht dargestellte Führungsmittel insbesondere an den Revolvern 21 und 22 wird eine Verdrehung des zweiten Revolvers 22 gegenüber den Revolvern 21 und 23 bewirkt. Hierfür kann insbesondere eine integrierte "Kugelschreibermechanik" eingesetzt werden. Durch mechanische Mittel an dem Revolver 21, insbesondere durch Dorne 27, wird ein Aufreißen von Kavitäten 28, 29 oder 30 in dem Revolver 21 bewirkt, je nach Position des Revolvers 22 im Verhältnis zu Revolver 21. Dies wird dadurch realisiert, dass die Kavitäten 28, 29 und 30 in ihrem in dieser Darstellung unteren Bereich mit einer durchstechbaren Folie ausgestattet sind. Durch Anreißen mit den Dornen 27 wird die in den jeweiligen Kavitäten 28, 29 und 30 enthaltene Flüssigkeit in Pfeilrichtung freigesetzt. Hierbei können beispielsweise in den Kavitäten 28 und 29 verschiedene Reagenzien enthalten sein. In der Kavität 30 kann beispielsweise ein Zelllysat als Probe vorgehalten werden, in der sich das aufzureinigende Molekül, beispielsweise ein Protein, befindet. Der zweite Revolver 22 weist eine zentrale Kavität als Inkubationskammer 31 auf. In diese Reaktionskammer werden die Probe, also das Lysat, sowie in Abhängigkeit von dem Aufreinigungsprotokoll verschiedene Reagenzien eingebracht. Weiterhin befinden sich die Beads 32 in der Inkubationskammer 31. Hier findet die Wechselwirkung zwischen den Beads 32 als Festphase und dem aufzureinigenden Molekül aus der flüssigen Probe statt. In Richtung des Fluidflusses ist die Reaktionskammer 31 in einem Bereich von einer Membran 111 begrenzt. Die Membran 111 kann beispielsweise einen Durchmesser von 1 bis 7 mm aufweisen, insbesondere in Anpassung an die Dimension der erfindungsgemäßen Vorrichtung. Erfindungsgemäß ist diese Membran 111 in Abhängigkeit von der wirkenden Zentrifugalkraft oder gleichwirkenden Kraft durchlässig oder undurchlässig für Flüssigkeiten, so dass die Membran 111 als "Ventil" genutzt werden kann, um die Inkubationskammer 31 für einen Durchlauf der Flüssigkeit öffnen zu können. Die Membran 111 ist nur in einem bestimmen abgegrenzten Bereich des Bodens der Inkubationskammer 31 vorgesehen. Der übrige Bereich ist für Flüssigkeiten nicht passierbar. Je nach Orientierung des Revolvers 22 befindet sich der durchlässige Bereich mit der Membran 111 in räumlicher Nähe bzw. oberhalb einer bestimmten Kavität des dritten Revolvers 23. In dieser Ausführungsform sind zwei Kavitäten im Revolver 23 vorhanden. Die Kavität 33 ist für Abfallflüssigkeit (waste) vorgesehen. Die Kavität 34 ist für die Aufnahme eines üblichen Reaktionsgefäßes, beispielsweise eines Eppendorf-Reagenzgefäßes vorgesehen, in dem ein Eluat aus der Inkubationskammer 31 aufgefangen werden kann, wobei das Eluat das gereinigte Molekül enthält. Eine Auswechselbarkeit des Reaktionsgefäßes in der Kavität 34 ermöglicht hierbei das Auffangen verschiedener Fraktionen aus dem Aufreinigungsprozess, die in geeigneter Weise analysiert und/oder weiterverarbeitet werden können.

Die erfindungsgemäße Vorrichtung 20 ist beispielsweise zur Durchführung einer Bead-basierten Proteinaufreinigung geeignet. Die Beads 32 befinden sich in der Inkubationskammer 31 und werden mit verschiedenen Lösungen und Reagenzien aus dem Revolver 21 beaufschlagt. Die Steuerung des Ablaufs erfolgt über eine Verdrehung des Revolvers 22 im Verhältnis zum Revolver 21 und zum Revolver 23, wobei durch die Dorne 27 die Reagenzien und übrigen Lösungen aus dem Revolver 21 in der gewünschten Weise in die Inkubationskammer 31 freigesetzt werden. Durch die Orientierung des Revolvers 22 im Verhältnis zum Revolver 23 wird die Flüssigkeit aus der Inkubationskammer 31 in die Kavitäten bzw. Gefäße im Revolver 23 überführt. Dieser Fluidfluss wird über die Membran 111 gesteuert, die als Ventil wirkt. Die Ansteuerung des "Ventils" erfolgt über die angelegte Zentrifugalkraft. Der generelle Protokollablauf ist hierbei, dass die Beads zunächst mit einer wässrigen Lösung zur Einstellung des geeigneten pH-Wertes an der Bead-Oberfläche umspült werden. Anschließend wird das Zelllysat, das das aufzureinigende Protein enthält, zugegeben und mit den Beads inkubiert, so dass eine spezifische Bindung der Proteine an die Beads erfolgt. Nach der Inkubation wird nicht gebundenes Lysat über die Membran 111 mit Waschpuffer ausgespült, bevor eine spezifische Elution mit einem Elutionspuffer erfolgt. Auf diese Weise kann beispielsweise ein His-tag-Protein, also ein rekombinantes Protein, das mit einem Histidin-Tag versehen ist, aus einem entsprechenden bakteriellen Lysat aufgereinigt werden. Hierfür können nicht magnetische Beads mit einem Durchmesser von beispielsweise > 5 pm, die mit Nickel versehen sind, eingesetzt werden, z.B. Ni-NTA-Beads. Die Beads werden aus einer der Vorlagerungskammern 28 oder 29 in die Inkubationskammer 31 überführt. Die Prozessierung der Aufreinigung erfolgt analog zu einem manuellen Protokoll, wobei die benötigten Flüssigkeiten in den Kavitäten des Revolvers 21 vorgelagert und je nach Bedarf freigesetzt und in die Inkubationskammer 31 überführt werden. Das Spülen bzw. Waschen und Eluieren der Beads erfolgt über die Ventilwirkung der Membran 111, wobei die Membran bei Gravitationsbeschleunigungen < 100 g die aufgegebene Flüssigkeit zurückhält und bei Gravitationsbeschleunigungen > 100 g die Flüssigkeit über der Membran vollständig durchlässt und kein Restvolumen in den Membranporen verbleibt. Die Inkubation der Flüssigkeiten kann im Ruhezustand der Zentrifuge geschehen oder bei geringen Gravitationsbeschleunigungen, insbesondere bei < 100 g.

Ein geeignetes Ablaufprotokoll ist in der folgenden Tabelle dargestellt.

| **Schritt** | **Aktion** | **Zeit** | **Zentrifugation** |
|---|---|---|---|
| Beladung mit den Beads | Zugabe von 100 µl Bead-Suspension Ausspülen | 60 s | 1500 g |
| Puffern | 250 µl Waschpuffer | 5 s | 10 g |
| | Inkubation | 55 s | 40 g |
| | Ausspülen | 60 s | 1500 g |
| Zugabe des Lysats | 500 µl Lysat | 5 s | 10 g |
| | Inkubation | 55 s / 29 min | 40 g / 10 g |
| | Ausspülen | 60 s | 6000 g |
| Waschen I | 250 µl Waschpuffer | 5 s | 10 g |
| | Inkubation | 55 s | 40 g |
| | Ausspülen | 60 s | 6000 g |
| Waschen II | 250 µl Waschpuffer | 5 s | 10 g |
| | Inkubation | 55 s | 40 g |
| | Ausspülen | 60 s | 6000 g |
| Waschen III | 250 µl Waschpuffer | 5 s | 10 g |
| | Inkubation | 55 s | 40 g |
| | Ausspülen | 60 s | 6000 g |
| Elution | 100 µl Elutionspuffer | 5 s | 10 g |
| | Inkubation | 55 s / 60 s | 40 g / 10 g |
| | Ausspülen | 60 s | 6000 g |

Ausgangspunkt für die Entwicklung eines geeigneten Ablaufprotokolls kann ein manuelles Protokoll sein, wobei die einzelnen Protokollschritte an die verwendeten Beads und die verwendete Membran angepasst werden.

Bei der Durchführung des beschriebenen Reinigungsprotokolls sollte beachtet werden, dass sich im Verlauf des Protokolls die Durchlässigkeit der Membran verändern kann, da das Porensystem der Membran durch die Zugabe des Lysats durch Partikel und Agglomerate aus dem Lysat zugesetzt werden kann. Dies kann zur Folge haben, dass sich die Rückhaltewirkung der Membran verstärkt.

Die erfindungsgemäße Vorrichtung ist im Prinzip für alle Arten von Beadbasierter Aufreinigung geeignet, beispielsweise können verschiedene Proteine, Peptide, DNA oder RNA aus einer Probe aufgereinigt werden. Hierfür ist lediglich die Auswahl von jeweils geeigneten Beads bzw. eine geeignete Vorbehandlung der Beads mit entsprechenden Wechselwirkungspartnern der jeweils aufzureinigenden Substanz und/oder eine Anpassung der Bindungschemien in an sich bekannter Weise erforderlich.

In anderen Ausgestaltungen der erfindungsgemäßen Vorrichtungen können weitere Membranen mit Ventilfunktion in der Vorrichtung eingesetzt werden, um den Fluidfluss auch zwischen anderen Kavitäten, beispielsweise zwischen Reagenzienvorhaltekavitäten und einer Inkubationskammer zu bewerkstelligen.

## Patentansprüche

1. Vorrichtung (20) zur Durchführung von chemischen und/oder biochemischen Prozessen, wobei die Vorrichtung (20) wenigstens zwei axial übereinander angeordnete Körper (21, 22, 23) mit jeweils wenigstens einer Kavität (28, 29, 30, 31, 33, 34) aufweist und wobei die Körper (21, 22, 23) in Abhängigkeit von einer Zentrifugalkraft oder einer gleichwirkenden Kraft gegeneinander verdrehbar sind, **dadurch gekennzeichnet, dass** in wenigstens einer Kavität (10; 31) eine Membran (11; 111) vorgesehen ist, deren Durchlässigkeit für Flüssigkeiten von der wirkenden Zentrifugalkraft oder der gleichwirkenden Kraft abhängig ist.

2. Vorrichtung nach Anspruch 1, **dadurch gekennzeichnet, dass** in der Kavität (10; 31) mit der Membran (11; 111) Beads (15; 32) vorhanden sind.

3. Vorrichtung nach Anspruch 1 oder Anspruch 2, **dadurch gekennzeichnet, dass** die Membran (11; 111) bei einer Gravitationsbeschleunigung von < 100 g für Flüssigkeiten undurchlässig und bei einer Gravitationsbeschleunigung von > 100 g für Flüssigkeiten durchlässig ist.

4. Vorrichtung nach einem der vorhergehenden Ansprüche, **dadurch gekennzeichnet, dass** die Porengröße der Membran (11; 111) 10 µm oder kleiner, insbesondere 5 µm oder kleiner, vorzugsweise 1 µm oder kleiner, ist.

5. Vorrichtung nach einem der vorhergehenden Ansprüche, **dadurch gekennzeichnet, dass** die Membran (11; 111) eine PVDF-Membran ist.

6. Vorrichtung nach einem der vorhergehenden Ansprüche, **dadurch gekennzeichnet, dass** die Membran (11) auf einer porösen Trägerstruktur (12) gelagert ist.

7. Vorrichtung nach einem der vorhergehenden Ansprüche, **dadurch gekennzeichnet, dass** für die Verdrehung der Körper (21, 22, 23) gegeneinander ein Eingriff von Führungsfedern des einen Körpers (22) in eine Profilzahnreihe (23) des anderen Körpers und eine entgegen der Zentrifugalkraft oder entgegen der gleichwirkenden Kraft wirkende Rückstellkraft der Körper vorgesehen sind.

8. Verwendung eines Reaktionsbehältnisses (10) mit einer darin angeordneten Membran (11), deren Durchlässigkeit für Flüssigkeiten von einer wirkenden Zentrifugalkraft oder einer gleichwirkenden Kraft abhängig ist, für die Durchführung von chemischen und/oder biochemischen Prozessen, insbesondere für die Durchführung einer automatisierten Reinigung von Proteinen und/oder von genetischem Material.

9. Verwendung nach Anspruch 8, **dadurch gekennzeichnet, dass** die chemischen und/oder biochemischen Prozesse Bead-basiert sind.

10. Verwendung nach Anspruch 8 oder Anspruch 9, **dadurch gekennzeichnet, dass** das Reaktionsbehältnis (10) Teil einer Vorrichtung (20) gemäß einem der Ansprüche 1 bis 7 ist.
